# EUROPEAN PATENT APPLICATION

(11) **EP 4 483 887 A1**
(43) Date of publication of application: **01.01.2025**
(21) Application number: 23774429.7
(22) Date of filing: 02.03.2023
(51) Int. Cl.: A61K 35/12, A61K 35/28, A61K 35/51, A61P 25/00, A61P 25/16, A61P 25/18, A61P 25/24, A61P 25/28, A61P 43/00, C12N 5/0735, C12N 5/074, C12N 5/0775, C12N 5/0789, C12N 5/0797, C12N 5/10

(54) **CELL PREPARATION**

(30) Priority: 23.03.2022 JP 2022046721
(71) Applicant: Foundation for Biomedical Research and Innovation at Kobe, Kobe-shi, Hyogo 650-0047 (JP)
(72) Inventor: TAGUCHI Akihiko, Kobe-shi, Hyogo 650-0047 (JP); OKINAKA Yuka, Kobe-shi, Hyogo 650-0047 (JP); OGAWA Yuko, Kobe-shi, Hyogo 650-0047 (JP); KIMURA Takafumi, Ibaraki-shi, Osaka 567-0085 (JP); TANAKA Mitsunobu, Ibaraki-shi, Osaka 567-0085 (JP); YASUI Kazuta, Ibaraki-shi, Osaka 567-0085 (JP); FUCHIZAKI Akihiro, Ibaraki-shi, Osaka 567-0085 (JP)
(74) Representative: Germain Maureau
(86) International application number: PCT/JP2023/007766
(87) International publication number: WO 2023/181830

(57) **Abstract**

Provided is a cell preparation that has an effective therapeutic effect on ischemic disease. The cell preparation has CD18-positive cells. The cell preparation is produced by obtaining a buffy coat layer from a sample containing hematopoietic stem cells, obtaining mononuclear cells by removing, from the buffy coat layer, inhibitory cells that inhibit a therapeutic effect, obtaining, from the mononuclear cells, CD34⁺ mononuclear cells that are CD34-positive, and obtaining, from the CD34-positive mononuclear cells, CD34⁺CD18^{high} mononuclear cells in which CD18 is highly expressed.

## Description

### TECHNICAL FIELD

The present invention relates to cell preparations and the like for treatment of diseases that can be expected to be cured by promoting new tissue formation.

### BACKGROUND ART

The formation of new tissue is crucial for the functional restoration of tissue damaged by ischemic disease. It has in recent years been found that cell transplantation is effective for such functional restoration. NON-PATENT DOCUMENT 1 indicates that for myocardial infarction, administration of bone marrow mononuclear cells obtained by specific gravity centrifugation allows formation of a new heart microvascular network, leading to an improvement in cardiac function. NON-PATENT DOCUMENT 2 indicates that for limb ischemia, administration of peripheral blood-derived CD34-positive cells purified using a specific cell-surface antigen allows formation of a new limb microvascular network, leading to an improvement in ischemia syndrome. NON-PATENT DOCUMENT 3 indicates that for limb ischemia, administration of bone marrow mononuclear cells obtained by specific gravity centrifugation allows formation of a new limb microvascular network, leading to an improvement in ischemia syndrome. NON-PATENT DOCUMENT 4 indicates that for cerebral infarction, administration of umbilical cord blood-derived CD34-positive cells purified using a specific cell-surface antigen allows formation of a new cerebral microvascular network, leading to an improvement in neurological function. NON-PATENT DOCUMENT 5 indicates that for cerebral infarction, administration of bone marrow mononuclear cells obtained by specific gravity centrifugation allows formation of a new cerebral microvascular network, leading to an improvement in neurological function.

Meanwhile, there are several problems with treatment of ischemic disease using autologous hematopoietic stem cells. For example, patients with acute ischemic cerebral infarction have unstable systemic conditions including the blood pressure, heart rate, respiratory state, and various physiological functions. In addition, the patient's level of consciousness is often low, and therefore, there typically are difficulties in collecting bone marrow cells. Therefore, it is difficult to perform the above-described treatment in situations where it is difficult to receive help from the anesthesiologist or hematologist, or in the case in which there is a high risk of deterioration of medical condition, occurrence of a harmful event, or the like involved with collection of autologous bone marrow fluid. Thus, for widespread use of ischemic disease treatment by stem cell transfusion, the use of allogeneic hematopoietic stem cells, for which collection, preparation, and preservation methods have been established, is considered to be ideal.

However, it is known that in application of allogeneic hematopoietic stem cells to treatment of ischemic disease, some of allogeneic hematopoietic stem cells exhibit an effective therapeutic effect, but others do not. NON-PATENT DOCUMENT 6 indicates that allogeneic hematopoietic stem cell transplantation may cause damage to a microvascular network, leading to symptomatic deterioration after myocardial infarction. NON-PATENT DOCUMENT 7 indicates that allogeneic hematopoietic stem cell transplantation may cause damage to a microvascular network, leading to symptomatic deterioration after cerebral infarction. Thus, in order to achieve the therapeutic effect of cell transplantation, particularly allogeneic cell transplantation, there is a demand for a cell preparation obtained by separating cells that exhibit a beneficial therapeutic effect from cells that do not, and isolating beneficial cells or removing harmful cells.

The importance of new formation is also suggested in the field of cerebral nerves. Although it had been considered that nerve cells do not grow in adult human and rodent brains, it was recently discovered that new neurons are constantly generated in the hippocampus (NON-PATENT DOCUMENTS 8 and 9).

Newborn neurons are incorporated into a nervous network and function therein. The frequency of the birth of neurons (neurogenesis) in the hippocampus increases during learning or in an enriched environment.

Conversely, however, it has been reported that neurogenesis decreases due to aging (NON-PATENT DOCUMENTS 10 and 11). The research group in University of Illinois at Chicago, The University of British Columbia, Rush University Medical Center reported that hippocampal neurogenesis persists even in adult humans, but drops sharply in aged humans, suggesting that neurogenesis correlates with cognitive conditions (NON-PATENT DOCUMENT 12).

It has been reported that neurogenesis in the brain also decreases in psychiatric disorders such as depression and schizophrenia, suggesting that such a decrease is involved with pathological conditions in these disorders. Hippocampal neurogenesis is inhibited by stress, which is considered to be a risk factor for development of depression, and is conversely promoted by chronic administration of antidepressants (NON-PATENT DOCUMENTS 13 and 14).

It has been reported that hippocampal neurogenesis decreases in Alzheimer's disease, suggesting that such a decrease is involved with pathological conditions in the disease. A study using mice having a genetically modified version of the amyloid precursor protein gene (APP), which is closely related to Alzheimer's disease, suggested that amyloid-β (Aβ) is involved with the reduced hippocampal neurogenesis.

Therefore, promotion of hippocampal neurogenesis would suggest the possibility of a novel method for treatment of an age-related decrease in neurological function, psychiatric disorders, and dementia.

### CITATION LIST

### NON-PATENT DOCUMENTS

NON-PATENT DOCUMENT 1: Autologous transplantation of bone marrow mononuclear cells improved heart function after myocardial infarction. Lin et al. Acta Pharmacol Sin 2004 Jul; 25 (7): 876-886
NON-PATENT DOCUMENT 2: Autologous transplantation of peripheral blood endothelial progenitor cells (CD34+) for therapeutic angiogenesis in patients with critical limb ischemia. Kudo et al. Int Angiol. 2003 Dec;22(4):344-8
NON-PATENT DOCUMENT 3: Therapeutic Angiogenesis by Autologous Bone-marrow Transplantation in a General Hospital Setting. Taguchi et al. Eur J Vasc Endovasc Surg. 2003 Mar;25(3):276-8
NON-PATENT DOCUMENT 4: Administration of CD34+cells after stroke enhances neurogenesis via angiogenesis in a mouse model. Taguchi et al. J Clin Invest. 2004 Aug; 114(3):330-8
NON-PATENT DOCUMENT 5: Bone marrow mononuclear cells promote proliferation of endogenous neural stem cells through vascular niches after cerebral infarction. Nakano-Doi et al. Stem Cells. 2010 Jul;28(7):1292-302
NON-PATENT DOCUMENT 6: A novel sialyl LewisX analog attenuates neutrophil accumulation and myocardial necrosis after ischemia and reperfusion. Lefer et al. Circulation. 1994 Nov;90(5):2390-401
NON-PATENT DOCUMENT 7: Polymorphonuclear leukocytes occlude capillaries following middle cerebral artery occlusion and reperfusion in baboons. del Zoppo et al. Stroke. 1991 Oct;22(10):1276-83
NON-PATENT DOCUMENT 8: Volume reduction in subcortical regions according to severity of Alzheimer's disease. Roh et al. Neurol. 2011. 258:1013-1020
NON-PATENT DOCUMENT 9: Dopamine depletion impairs precursor cell proliferation in Parkinson disease. Hoglinger et al. Nat. Neurosci. 2004. 7: 726-735
NON-PATENT DOCUMENT 10: Hippocampal atrophy in recurrent major depression. Sheline et al. Proc. Natl. Acad. Sci. USA 1996. 93: 3908-3913
NON-PATENT DOCUMENT 11: Neural stem cell proliferation is decreased in schizophrenia, but not in depression. Reif et al. Mol. Psychiatry 2006. 11: 514 - 522
NON-PATENT DOCUMENT 12: Human Hippocampal Neurogenesis Persists in Aged Adults and Alzheimer's Disease Patients. Tobin et al. Cell Stem Cell 2019. 24, 974-982.e1-e3
NON-PATENT DOCUMENT 13: Adult neurogenesis in humans. Bergmann et al. Cold Spring Harb Perspect Biol. 2015 Jul 1;7(7):a018994.
NON-PATENT DOCUMENT 14: Neurogenesis in the striatum of the adult human brain. Ernst et al. Cell 2014. 156, 1072-1083
NON-PATENT DOCUMENT 15: Comparison of the value of PCNA and Ki-67 as markers of cell proliferation in ameloblastic tumors. Bologna-Molina et al. Med. Oral Patol. Oral Cir. Bucal 2013. 18, e174-e179

### SUMMARY OF THE INVENTION

### TECHNICAL PROBLEM

With the above problem in mind, the present invention has been made. It is an object of the present invention to provide a cell preparation having an effective therapeutic effect for diseases that can be expected to be cured by promoting new tissue formation.

### SOLUTION TO THE PROBLEM

A cell preparation according to the present invention has CD18-positive cells.

### ADVANTAGES OF THE INVENTION

According to the present invention, a cell preparation having an effective therapeutic effect on ischemic disease is obtained. In addition, according to the present invention, neurogenesis can be effectively promoted in the hippocampus.

### BRIEF DESCRIPTION OF THE DRAWINGS

[FIG. 1] FIG. 1 is a diagram for comparing a therapeutic effect exhibited when aggregates exist in umbilical cord blood mononuclear cells with a therapeutic effect exhibited when aggregates do not exist in umbilical cord blood mononuclear cells.
[FIG. 2] FIG. 2 is a diagram for comparing a therapeutic effect exhibited when umbilical cord blood mononuclear cells are subjected to a heparin treatment with a therapeutic effect exhibited when umbilical cord blood mononuclear cells are subjected to an ACD treatment.
[FIG. 3] FIG. 3 is a diagram showing changes in expression of various adhesion factors involved with cell-cell junctions before and after a heparin treatment.
[FIG. 4] FIG. 4 is a diagram showing a relationship between a therapeutic effect and CD18 for umbilical cord blood mononuclear cells.
[FIG. 5] FIG. 5 is a diagram showing a relationship between a therapeutic effect and connexin 43 or GLUT1.
[FIG. 6] FIG. 6 is a diagram showing changes in expression of CD18 before freezing and after thawing in umbilical cord blood mononuclear cells.
[FIG. 7] FIG. 7(A) is a diagram showing cell surface marker analysis by FACS in which at least 90% of living cells are CD18-positive, and FIG. 7(B) is a diagram showing cell surface marker analysis by FACS in which at least 60% of living cells are CX43-positive.
[FIG. 8] FIG. 8 is a diagram showing that Jagged1 is expressed in CD34+ cells.
[FIG. 9] FIG. 9 is a diagram showing that VEGF uptake performed by HUVECs is promoted by a stimulus exerted by Jagged1.
[FIG. 10] FIG. 10 is a diagram showing a quantitative assessment of the number of newborn neurons in the hippocampal dentate gyrus, demonstrating that both nestin-positive cells (A) and DCX-positive cells (B) are statistically significantly increased due to administration of CD18-positive cells.

### DESCRIPTION OF EMBODIMENTS

Embodiments of the present invention will be specifically described below with reference to the accompanying drawings. The embodiments are for the purpose of facilitating understanding of the principle of the present invention. The scope of the present invention is not intended to be limited to the embodiments below. Those skilled in the art will make substitutions to the embodiments when necessary without departing from the scope of the present invention.

A cell preparation according to the present invention is characterized by having CD18-positive cells. The CD18-positive cells are preferably a stem cell.

### (1) Stem cells

Stem cells have the ability to differentiate into various cells, and the self-renewal ability. Examples of stem cells include adult stem cells and pluripotent stem cells.

Adult stem cells exit in adult tissues, and are not fully differentiated and have pluripotency to some extent. Examples of adult stem cells include hematopoietic stem cells, mesenchymal stem cells, neural stem cells, intestinal epithelial stem cells, hair follicle stem cells, and pigment stem cells.

Pluripotent stem cells have pluripotency, which is the ability to differentiate into all types of cells constituting an organism, and can continue to proliferate endlessly while maintaining pluripotency when cultured in vitro under appropriate conditions. Examples of pluripotent stem cells include embryonic stem cells (ES cells), embryonic germ stem cells (EG cells), germline stem cells (GS cells), and artificial pluripotent stem cells (iPS cells).

In the cell preparation according to the present invention, the stem cell is preferably a hematopoietic stem cell.

Hematopoietic stem cells have the pluripotency to differentiate into all blood cell lineages of blood cells, and the self-renewal ability while maintaining the pluripotency.

Hematopoietic stem cells are isolated from the above-described tissue, typically using expression of a surface marker specific to the cells (hematopoietic stem cell specific marker). Concerning expression of such a marker, human-derived hematopoietic cells are typically characterized by being CD34-positive. More specifically, hematopoietic stem cells are a CD34-positive (+), CD38-negative (-), CD90-positive (+), and CD45RA-negative (-) cell, preferably a CD34⁺CD38⁻CD90⁺CD45RA⁻CD49f⁺ cell.

Tissues from which hematopoietic stem cells are derived are not particularly limited as long as hematopoietic stem cells exist therein. The tissue is preferably hematopoietic tissue. Examples of the tissue include bone marrow, umbilical cord blood, peripheral blood, and liver.

CD34-positive cells mean CD34-positive enriched cells, which can, for example, be obtained by concentrating CD34-positive cells from a sample of bone marrow, peripheral blood, placenta, umbilical cord blood, or the like using a technique well known in the art. CD34-positive cells can, for example, be concentrated using CD34-immobilized beads or a cell sorter. A preferable example of CD34-positive cells is a CD34-positive mononuclear cell.

The hematopoietic stem cells in the present invention are allogeneic cells in light of medical convenience, but do not exclude autologous cells. In an embodiment, the hematopoietic stem cells are prepared from an allogeneic umbilical cord blood sample that is a pharmaceutical composition according to the present invention. In another embodiment, the hematopoietic stem cells are prepared from a banked cell group.

### (2-1) CD18

CD18 is also called β2 integrin. Integrins are molecules that have a key role in cell adhesion and are expressed in various cells. Integrins are transmembrane proteins that are a dimer composed of α and β chains. Twenty-four integrins are each composed of one of 18 α chains and one of 8 β chains.

CD18 is also called leukocyte integrin, and the expression thereof is specific to leukocytes. However, the present inventors paid attention to the fact that CD18 is also expressed in hematopoietic stem cells and the like in addition to leukocytes to find that CD18 has a key role in cell adhesion of hematopoietic stem cells. CD18 is normally incapable of binding to a ligand, and when activated, undergoes a reversible three-dimensional structural change to be able to bind to a ligand.

CD18-positive cells are a cell group in which the percentage of cells having a CD18-positive reaction in, for example, analysis by flow cytometry is at least 60%, preferably at least 70%, more preferably at least 80%, and even more preferably at least 90%.

CD18-positive cells may, for example, also be a cell group in which, in a flow cytometry assay, the median intensity of fluorescence (MIF) of a CD18 antigen can be maintained at least two times, preferably at least three times, and more preferably at least four times as high as an MIF obtained when reacted with an isotype control antibody.

### (2-2) Glucose transporters (GLUTs)

Glucose transporters (GLUTs) are membrane proteins on the plasma membrane that are required for transport of glucose across the biological membrane, and are called a glucose transporting carrier. At present, 13 GLUTs have been reported (GLUT-1, -2, -3, -4, -5, -6, - 7, -8, -9, -10, -11, and -12, and HMIT). For four of them, i.e., GLUT1-GLUT4, which belong to class I, the expression sites and roles thereof have been relatively well clarified. GLUT1-GLUT4 all have a molecular weight of about 50 kDa and share the same structure in which the protein spans the cell membrane 12 times. A class-I glucose transporter (GLUT1) has a key role in transport of glucose from cerebral blood vessels to the brain. The present inventors found that GLUT1 is deeply involved with exhibition of the effect of hematopoietic stem cells.

### (2-3) Connexin 43

Connexin is a generic term for a family of junction membrane proteins forming gap junctions. More than 12 subtypes of connexins having different molecular weights have been found. A connexin having a molecular weight of 26 kDa is called connexin 26, and a connexin having a molecular weight of 43 kDa is called connexin 43. Connexin 43 is more abundant in the central nervous system and cardiomyocytes than are the other members of the connexin family, and is presumed to have adverse effects such as arrhythmias when the amount thereof is reduced. The present inventors found that connexin 43 is deeply involved with exhibition of the effect of hematopoietic stem cells.

### (2-4) Jagged1

Jagged1 is a ligand for a Notch receptor. The Notch signaling pathway is very important to cell-cell communication. The present inventors found that Jagged1 is deeply involved with exhibition of the effect of hematopoietic stem cells.

(2-5)
Thus, the blood vessel regeneration promotion mechanism of hematopoietic stem cells provides a simple cell-cell interaction (interaction between hematopoietic stem cells and damaged blood vessel endothelial cells) immediately after administration of the cells, and does not require engraftment, proliferation, or differentiation of the transplanted stem cells. If CD18 is expressed, the administered hematopoietic stem cells effectively adhere to blood vessel endothelial cells. If a connexin is expressed, the administered hematopoietic stem cells are effectively linked to blood vessel endothelial cells through gap junctions. If a glucose transporter (GLUT) is expressed, blood vessel endothelial cells are effectively supplied with energy from hematopoietic stem cells, which have abundant glucose, through gap junctions. If Jagged1 is expressed, cell-cell communication is effectively established between the administered hematopoietic stem cells and blood vessel endothelial cells.

Therefore, a cell preparation that can be expected to have an excellent therapeutic effect is characterized by having CD18-positive cells. In the CD18-positive cells, a glucose transporter GLUT (particularly preferably GLUT1) is preferably expressed. In the CD18-positive cells, a connexin (particularly preferably connexin 43) is preferably expressed. In the CD18-positive cells, Jagged1 is preferably expressed.

In addition, as described below, the expression intensity of a glucose transporter (GLUT) as an indicator of VEGF uptake promotion capability is useful as a marker for selecting mononuclear cells having a high therapeutic effect. Therefore, a cell preparation that can be expected to have an excellent therapeutic effect is characterized by having GLUT-positive cells (preferably GLUT1-positive cells).

In addition, as described below, the expression intensity of a connexin as an indicator of VEGF uptake promotion capability is useful as a marker for selecting mononuclear cells having a high therapeutic effect. Therefore, a cell preparation that can be expected to have an excellent therapeutic effect is characterized by having a connexin-positive cell (preferably connexin 43-positive cell).

In addition, as described below, it has been demonstrated that expression of Jagged1 in stem cells is important to regeneration promotion effects. Therefore, a cell preparation that can be expected to have an excellent therapeutic effect is characterized by having Jagged1-positive cells.

### (3) Cell preparation

The cell preparation according to the present invention is for treatment and/or prevention of diseases that can be expected to be cured by new tissue formation. As used herein, "treatment" includes treatment of syndromes, improvement of syndromes, and inhibition of progression of syndromes. Meanwhile, "prevention" includes inhibition and slowing of the onset of diseases, and includes not only prevention of diseases before the onset thereof but also prevention of the recurrence of diseases after the treatment thereof.

The cell preparation according to the present invention is also for ischemic diseases such as cerebral infarction, myocardial infarction, limb ischemia, renal infarction, pulmonary infarction, splenic infarction, and intestinal infarction. The cell preparation according to the present invention is intended to cause formation of new microvessels around the ischemic area by administering stem cells in which CD18 is highly expressed, thereby overcoming ischemic diseases. Diseases targeted by the cell preparation according to the present invention are not limited to those described in the examples.

In the case in which the cell preparation according to the present invention is used for cerebral infarction, the cell preparation according to the present invention can be used in any of the hyperacute, acute, subacute, and chronic phases. In particular, the cell preparation according to the present invention is suitably used in the acute and subacute phases for acute-onset ischemic diseases such as acute myocardial infarction and cerebral infarction. Here, the hyperacute phase is within 12 hours from the onset, in which tissue cell death can be highly likely to be prevented by stenting, thrombolysis, thrombectomy, or the like. The acute phase is within 12 hours to 7 days from the onset. The subacute phase is within 1 to 4 weeks from the onset. The chronic period is at least one month from the onset. Meanwhile, the cell preparation according to the present invention can be used suitably for ischemic diseases that follow a chronic course, such as limb ischemia caused by chronic circulatory disorders, regardless of timing.

The cell preparation according to the present invention also targets diseases to whose treatment hippocampal neurogenesis contributes. Specifically, the cell preparation according to the present invention is a neurogenesis promotion agent that is characterized by having CD18-positive cells and allows hippocampal neurogenesis.

It has in recent years been clarified that neural stem and progenitor cells exist in the hippocampus, which is a specific brain region, even during the period of maturity, and that these cells grow and differentiate to produce new neurons. Newborn neurons play a key role in formation of neural networks, memory generation, and the like.

It has however been suggested that aging inhibits neurogenesis in the hippocampus, which is partly responsible for a decrease in neurological function of the elderly.

Stress is considered to be a risk factor for the development of psychiatric disorders such as depression. In animals, hippocampal neural stem and progenitor cells are reduced not only when the animal is physically and mentally stressed by, for example, restraint stress, but also when the animal is only mentally stressed by, for example, resident-intruder stress. This suggests that inhibition of hippocampal neurogenesis may be involved with the causes of psychiatric disorders such as depression.

It has been reported that dementia associated with neurodegenerative diseases has a decrease in neurogenesis in the hippocampus, suggesting that such a decrease is involved with the pathological conditions of the disease.

Therefore, promotion of neurogenesis in the hippocampus allows prevention and/or treatment of an age-related decrease in neurological function, and prevention and/or treatment of diseases associated with an age-related impairment of neurological function. Furthermore, promotion of neurogenesis in the hippocampus allows prevention and/or treatment of dementia associated with neurodegenerative diseases, and prevention and/or treatment of psychiatric disorders.

The hippocampus is located in the medial temporal lobe of the cerebrum at the base of the inferior horn of the lateral ventricle, and is a cortical site essential for the formation of explicit memory such as episodic memory. The dentate gyrus is an appendage of the hippocampus, but is herein considered to be part of the hippocampus. The dentate gyrus consists of three layers: a granule cell layer as a middle layer; a molecular layer located immediately above the middle layer and having a considerably low cell density; and a polymorphic cell layer located below the middle layer and appearing to have sparsely scattered cells.

The CD18-positive cells are preferably connexin 43-positive. All tissues and organs in the adult body have various forms of junctions between adjacent cells to maintain tissue homeostasis. Connexin 43, which is a molecule having a key role in cell-cell gap junctions, is expressed in, for example, osteoblasts, osteocytes, endovascular cells, and neurons. The present inventors found that connexin 43-positive cells have a key role in neurogenesis in the hippocampus (especially, neurogenesis in the dentate gyrus). Connexin 43-positive cells are a group of cells in which connexin 43 is highly expressed. The high expression of connexin 43 is not particularly limited. For example, if the percentage of cells in a cell group exhibiting a connexin 43-positive reaction as analyzed by flow cytometry analysis is at least 60%, preferably at least 70%, more preferably at least 80%, and even more preferably at least 90%, it is considered that connexin 43 is highly expressed in the cell group.

The administration route of the cell preparation according to the present invention is preferably intravenous, intraarterial, intraportal, or local tissue. The number of cells to be administered is not particularly limited and is determined, as appropriate, according to the age, weight, disease, and symptom of the target patient, and the technique of administration. For example, in the case of limb ischemia, the pharmaceutical composition according to the present invention is prepared such that hematopoietic stem cells are administered at a rate of about 10⁴ to 10⁶ cells/kg, preferably at least 10⁴ cells/kg, more preferably at least 5 × 10⁴ cells/kg, more preferably at least 5 × 10⁵ cells/kg, and optionally 2 × 10⁶ cells. In the case of cerebral infarction, the pharmaceutical composition according to the present invention is prepared such that hematopoietic stem cells are administered at a rate of about 10⁴ to 10⁶ cells/kg, preferably at least 10⁴ cells/kg, and more preferably about 5 × 10⁴ cells/kg. The total number of cells (mononuclear cells) is about 100 times as large as the numerical value of the hematopoietic stem cells.

In the present invention, an angiogenesis promotion factor can also be included. The angiogenesis promotion factor is not particularly limited. As the angiogenesis promotion factor, for example, VEGF, angiopoietins, PDGF, TGF-β, FGF, PlGF, matrix metalloproteinases, plasminogen activators, and the like, preferably angiopoietins, can be used. Angiopoietins, which are glycoproteins, are growth factors that promote vasculogenesis or angiogenesis. Angiopoietins include angiopoietin 1 (Ang1), angiopoietin 2 (Ang2), angiopoietin 3 (Ang3), and angiopoietin 4 (Ang4). There are six angiopoietin-related proteins that are similar to angiopoietins (ANGPTLs: angiopoietin-related proteins or angiopoietin- like proteins).

When the cell preparation according to the present invention is used in the form of an injection preparation, additives that are commonly used in the art can be used if necessary. Examples of the additives include tonicity agents, stabilizing agents, buffering agents, preserving agents, chelating agents, antioxidants, and solubilizing agents. Examples of the tonicity agents include sugars, such as glucose, sorbitol, and mannitol, sodium chloride, glycerol, propylene glycol, and polyethylene glycol. Examples of the stabilizing agents include sodium sulfite. Examples of the buffering agents include borate buffers, phosphate buffers, citrate buffers, tartrate buffers, and acetate buffers. Examples of the preserving agents include paraoxybenzoic acid esters, benzyl alcohol, chlorocresol, phenethyl alcohol, and benzethonium chloride. Examples of the chelating agents include disodium EDTA and sodium citrate. Examples of the antioxidants include sodium sulfite, sodium hydrogen sulfite, sodium ascorbate, and sodium thiosulfate. Examples of the solubilizing agents include dextran, polyvinyl pyrrolidone, sodium benzoate, ethylenediamine, salicylamide, nicotinamide, and polyoxyethylene hydrogenated castor oil derivatives.

### (4) Method for producing cell preparation

In the case of a cell preparation having CD18 high expression cells, the cell preparation is produced by a method described below, although the present invention is not limited to the method.

The method has, for example: obtaining a buffy coat layer from a sample containing hematopoietic stem cells; obtaining mononuclear cells by removing, from the buffy coat layer, inhibitory cells that inhibit a therapeutic effect; and obtaining, from the mononuclear cells, CD18^{high} mononuclear cells in which CD18 is highly expressed.

The method also has, for example: obtaining a buffy coat layer from a sample containing hematopoietic stem cells; obtaining mononuclear cell cells by removing, from the buffy coat layer, inhibitory cells that inhibit a therapeutic effect; obtaining, from the mononuclear cells, CD34⁺ mononuclear cells that are CD34-positive; and obtaining, from the CD34-positive mononuclear cells, CD34⁺CD18^{high} mononuclear cells in which CD18 is highly expressed.

The method also has, for example: selecting a sample that contains a large number of CD18 high expression cells from a plurality of blood samples collected from a plurality of patients; and removing, from the blood samples, inhibitory cells that inhibit a therapeutic effect.

The method also has, for example: increasing CD18 high expression cells by causing a cytokine such as TNFα to act with the collected blood samples; and removing, from the blood samples in which CD18 high expression cells have been increased, inhibitory cells that inhibit a therapeutic effect.

In general, umbilical cord blood, blood, and bone marrow fluid are prone to coagulate, and therefore, heparin is often used as an anticoagulant. In the present invention, however, heparin is not used, and coagulation is inhibited by dilution with physiological saline and use of a divalent ion chelating agent.

As the sample containing hematopoietic stem cells, an umbilical cord blood, bone marrow fluid, or peripheral blood sample, preferably an umbilical cord blood sample, can be used.

For an umbilical cord blood sample, mononuclear cells that are accumulated in a buffy coat are obtained by density gradient centrifugation. In density gradient centrifugation, for example, commercially available media for density gradient centrifugation, such as Ficoll (registered trademark) and Percoll (registered trademark), are preferably used.

The buffy coat may contain inhibitory cells that inhibit the efficacy of the therapeutic effect of the cellular preparation. Therefore, it is desirable to remove such inhibitory cells from the buffy coat. Such inhibitory cells in the buffy coat are neutrophils and dead cells.

To remove the inhibitory cells from the buffy coat, an equal amount of PBS is added to the buffy coat layer, followed by centrifugation, to separate the inhibitory cells. The upper limit of centrifugal force for separating the inhibitory cells is, for example, 1000 g, 900 g, 800 g, 700 g, or 600 g, and the lower limit thereof is 500 g or 400 g. The centrifugal force is, for example, in the range of 400-1000 g, 400-900 g, 400-800 g, 400-700 g, or 400-600 g. The centrifugal force is preferably 400-800 g, more preferably 500-800 g.

The precipitate in the buffy coat layer after centrifugation has an upper layer that is a fraction in which neutrophils and dead cells are concentrated, and a lower layer that is a fraction in which mononuclear cells are concentrated. During discarding of the supernatant after centrifugation, neutrophils and dead cells in the upper layer of the precipitate are selectively removed by gradually tilting the container toward the horizontal. In addition, centrifugation under the same conditions and then passing through a 40-µm filter is desirably performed in order to remove neutrophils and dead cells from the mononuclear cell fraction.

Next, hematopoietic stem cells are obtained from the mononuclear cells in the buffy coat. Hematopoietic stem cells are obtained by concentrating CD34-positive cells according to a known technique. The concentration of CD34-positive cells can be performed using CD34-immobilized beads or a cell sorter.

Next, CD18-positive cells are obtained from the CD34-positive mononuclear cells in the buffy coat by concentration. The concentration of CD18-positive cells can be performed using CD18-immobilized beads or a cell sorter to obtain cells in which CD18 is highly expressed.

Thus, CD34⁺CD18^{high} mononuclear cells are obtained. It should be noted that the order of CD34⁺CD18^{high} is not limited to this embodiment. Cells in which CD18 is highly expressed may be obtained from mononuclear cells in the buffy coat before CD34-positive cells may be concentrated from the CD18 high expression cells.

### Examples

### (1) Influence of aggregates in umbilical cord blood mononuclear cell treatment

It has been found that in treatment of cerebral infarction using bone marrow mononuclear cells, the effect of a cerebral infarction treatment is significantly reduced in the case of a lot in which aggregates exist. Therefore, an experiment was conducted to see if a similar phenomenon occurs for umbilical cord blood mononuclear cells.

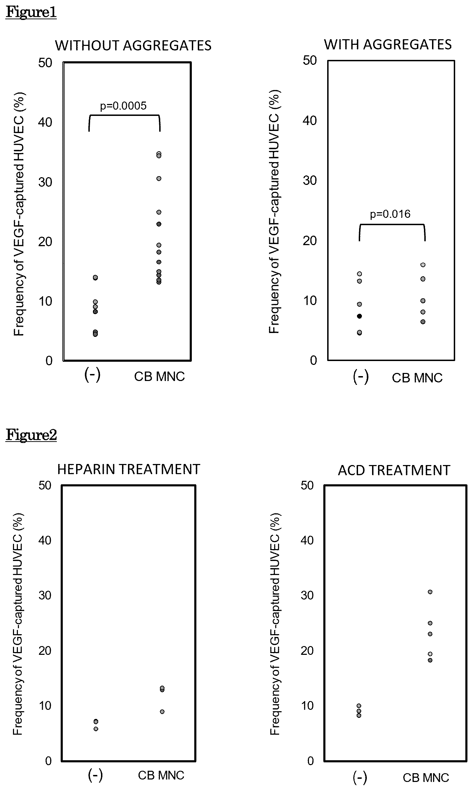

Mononuclear cells were purified from each of umbilical cord blood in which aggregates did not exist and umbilical cord blood in which large aggregates existed, by Ficoll-Paque specific gravity centrifugation, were washed with PBS, and thereafter, were co-cultured with HUVECs (FIG. 1). It should be noted that in FIG. 1, CBMNC indicates human umbilical cord blood-derived mononuclear cells.

A co-culture test was conducted as follows. Specifically, umbilical cord blood was gently placed on an equal amount of Ficoll-Paque, followed by centrifugation at 400 g for 30 minutes. At this time, the acceleration and deceleration of the centrifuge were set to the minimum values. A buffy coat layer was removed from the umbilical cord blood specimen after centrifugation. An equal amount of a PBS containing a 10% anticoagulant-citrate-dextrose solution (ACD solution) was added to the buffy coat layer, followed by centrifugation again at 400 g for 5 minutes. At this time, it is not necessary to set the acceleration and deceleration of the centrifuge to the minimum values. The precipitate in the buffy coat layer obtained by centrifugation had an upper layer that was a fraction in which neutrophils and dead cells were concentrated, and a lower layer that was a fraction in which mononuclear cells were concentrated. Therefore, during discarding of the supernatant, neutrophils and dead cells in the upper layer of the precipitate were selectively removed by gradually tilting the container toward the horizontal. Furthermore, the precipitate was centrifuged at 400 g for 5 minutes and was passed through a 40-µm filter to remove neutrophils and dead cells contained in the mononuclear cell fraction. Next, HUVECs were cultured in a HuMedia-EB2 culture medium with serum and growth additives according to the manufacturer's protocol, and thereafter, were dissociated with trypsin and recovered. An allophycocyanin (APC)-labeled vascular endothelial growth factor (VEGF, final concentration: 3 µg/mL) and the above-mentioned umbilical cord blood mononuclear cells (1 × 10⁶ cells) were added to the recovered HUVECs (1 × 10⁵ cells), followed by incubation in a 24-well plate in 5% CO₂ at 37°C for 3 hours. The cell mixture was washed twice with phosphate buffered saline (PBS), and was stained with a phycoerythrin (PE)-conjugated anti-human CD31 antibody (BD Bioscience), a fluorescein isothiocyanate (FITC)-conjugated anti-human CD45 antibody, and 7-aminoactinomycin D (7AAD). The level of APC-labeled VEGF taken into vascular endothelial cells (CD31-positive, CD45-negative, and 7AAD-negative) was assessed using a FACS Canto II cell analyzer (BD Bioscience). Since vascular endothelial cells undergoing angiogenesis take up VEGF from outside the cell, it is considered that umbilical cord blood having a higher APC-labeled VEGF level more strongly promotes angiogenesis, or in other words, has a more effective therapeutic effect on cerebral infarction.

As shown in FIG. 1, in the case of CBMNCs that did not undergo aggregation, vascular endothelial cells took up more VEGF due to the presence of CBMNCs. However, in the case of CBMNCs that underwent aggregation, vascular endothelial cells hardly took up VEGF even in the presence of CBMNCs. Thus, it was found that in none of umbilical cord blood mononuclear cells that undergo aggregation strongly promotes the uptake of VEGF, as with bone marrow mononuclear cells.

### (2) Decrease in therapeutic effect due to heparin treatment

In a co-culture test, a PBS prepared to contain heparin at a final concentration of 2 units/ml was erroneously added, instead of a PBS containing a 10% anticoagulant-citrate-dextrose solution (ACD solution).

Specifically, umbilical cord blood was gently placed on an equal amount of Ficoll Paque, followed by centrifugation at 400 g for 30 minutes. At this time, the acceleration and deceleration of the centrifuge were set to the minimum values. A buffy coat layer was removed from the umbilical cord blood specimen after centrifugation. A PBS prepared to contain heparin at a final concentration of 2 units/mL was erroneously added to the buffy coat layer, followed by centrifugation again at 400 g for 5 minutes, so that the buffy coat layer underwent precipitation. In addition, centrifugation was performed at 400 g for 5 minutes, followed by filtration through a 40-µm filter, in order to remove neutrophils and dead cells from the mononuclear cell fraction. Umbilical cord blood mononuclear cells thus obtained were subjected to the co-culture test described above.

In the case of the heparin treatment, a decrease in VEGF uptake similar to that observed when aggregates occurred was observed in the co-culture test using umbilical cord blood mononuclear cells, compared to the case of a normal PBS supplemented with an ACD solution (FIG. 2).

### (3) Discovery of importance of adhesion factor

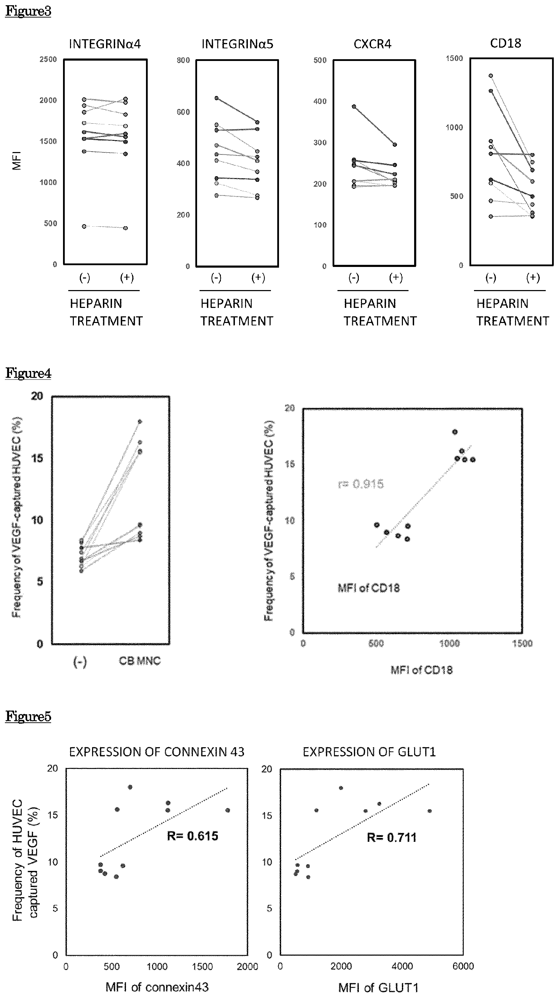

From the result shown in FIG. 2, it is considered that cell-cell junctions between hematopoietic stem cells and HUVECs are important. Therefore, changes in expression of various adhesion factors involved in cell-cell junctions, in hematopoietic stem cells, before and after the heparin treatment were compared (FIG. 3).

Specifically, umbilical cord blood was placed on an equal amount of Ficoll-Paque, followed by centrifugation at 400 g for 30 minutes. A buffy coat layer was removed from the umbilical cord blood specimen after centrifugation. An equal amount of a PBS containing a 10% anticoagulant-citrate-dextrose solution (ACD solution) was added, followed by centrifugation again at 400 g for 5 minutes. The precipitate in the buffy coat layer was separated by gradually tilting the container toward the horizontal and thereby discarding the supernatant. The precipitate was resuspended in a PBS, followed by centrifugation at 400 g for 5 minutes, and then filtration through a 40-µm filter. After filtration, the mononuclear cell suspension was equally divided, followed by centrifugation at 400 g for 5 minutes. After discarding of the supernatant, one of the two precipitates was suspended in a PBS, and the other was suspended in a PBS prepared to contain heparin at a final concentration of 2 units/mL, followed by incubation at room temperature for 5 minutes. Next, the cell suspensions were washed with a PBS, and were stained with a phycoerythrin (PE)-conjugated anti-human CD34 antibody (BD Bioscience), 7-aminoactinomycin D (7AAD) (BD Bioscience), fluorescein isothiocyanate (FITC)-conjugated anti-human CD45 antibody (BD Bioscience), or various adhesion factor antibodies (an integrin α4 antibody (Beckman Coulter), an integrin α5 antibody (Beckman Coulter), a CD18 antibody (Beckman Counter), or a CXCR4 antibody (MBL)) conjugated with allophycocyanin (APC). The expression levels of various adhesion factors in hematopoietic stem cells (CD34-positive and 7AAD-negative) were assessed with a FACS Canto II cell analyzer (BD Bioscience).

As shown in FIG. 3, the heparin treatment did not cause an observed significant change in expression of integrin α4, which is a representative adhesion factor that functions in hematopoietic stem cell homing and cell adhesion.

The adhesion factors whose expression was significantly changed due to the heparin treatment were integrin α5, C-X-C chemokine receptor type 4 (CXCR4), and CD18. Some of these molecules underwent a large reduction in expression, and others underwent almost no reduction in expression. In particular, it was CD18 that underwent a large change in expression due to the heparin treatment.

### (4) Discovery of a selectable marker for mononuclear cells having high therapeutic effect

Thus, some umbilical cord blood mononuclear cells that strongly promote VEGF uptake and others that do not were observed. A relationship between expression of CD18 in these cord blood hematopoietic stem cells and the cells' ability to promote VEGF uptake was studied to find a strong positive correlation between the two (FIG. 4).

Specifically, umbilical cord blood was placed on an equal amount of Ficoll-Paque, followed by centrifugation at 400 g for 30 minutes. A buffy coat layer was removed from the umbilical cord blood specimen after centrifugation. An equal amount of a PBS containing a 10% anticoagulant-citrate-dextrose solution (ACD solution) was added, followed by centrifugation again at 400 g for 5 minutes. The precipitate in the buffy coat layer was separated by gradually tilting the container toward the horizontal and thereby discarding the supernatant. The precipitate was resuspended in a PBS, followed by centrifugation at 400 g for 5 minutes, and then filtration through a 40-µm filter. The resultant precipitate was suspended again in a PBS. Next, aliquots of the suspension were removed and stained with a PE-conjugated anti-human CD34 antibody, 7AAD, an FITC-conjugated anti-human CD45 antibody, and an APC-conjugated CD18 antibody.

Furthermore, the remaining suspension was used to conduct a co-culture test with HUVECs (see (1) above). The co-culture experiment was conducted under a condition that an allophycocyanin (APC)-labeled vascular endothelial growth factor (VEGF, final concentration: 3 µg/mL) and umbilical cord blood mononuclear cells (cell count including 1 × 10⁴ CD34-positive cells) were added to the HUVECs (1 × 10⁵ cells).

The left portion of FIG. 4 shows differences in the VEGF uptake capability of HUVECs between each co-cultured umbilical cord blood. The right portion of FIG. 4 shows a correlation between expression of CD18 in hematopoietic stem cells (CD34-positive and 7AAD-negative) and the VEGF uptake capability of HUVECs in the co-culture experiment. It was found that the expression intensity of CD18 as an indicator of the VEGF uptake promotion capability, which was found by an accidental heparin treatment, i.e., an erroneous operation, is useful as a selectable marker for mononuclear cells having a high therapeutic effect.

Alternatively, in the above-mentioned experiment, the precipitate in the buffy coat layer was resuspended in a PBS, followed by centrifugation at 400 g for 5 minutes and then filtration through a 40-µm filter, and then resuspension in a PBS. Next, aliquots of the suspension were removed and stained with a PE-conjugated anti-human CD34 antibody, 7AAD, an FITC-conjugated anti-human CD45 antibody, and an APC-conjugated glucose transporter (GLUT1) antibody. In addition, the remaining suspension was used to conduct a co-culture test with HUVECs (see (1) above). The co-culture experiment was conducted under a condition that an allophycocyanin (APC)-labeled vascular endothelial growth factor (VEGF, final concentration: 3 µg/mL) and umbilical cord blood mononuclear cells (cell count including 1 × 10⁴ CD34-positive cells) were added to HUVECs (1 × 10⁵ cells). The right portion of FIG. 5 shows a correlation between expression of a glucose transporter (GLUT1) in hematopoietic stem cells (CD34-positive, CD45-positive, and 7AAD-negative) and the VEGF uptake capability of HUVECs in the co-culture experiment. It was found that the expression intensity of a glucose transporter (GLUT1) as an indicator of VEGF uptake promotion capability is useful as a selectable marker for mononuclear cells having a high therapeutic effect.

Alternatively, in the above-mentioned experiment, the precipitate in the buffy coat layer was resuspended in a PBS, followed by centrifugation at 400 g for 5 minutes and then filtration through a 40-µm filter, and then resuspension in a PBS. Next, aliquots of the suspension were removed and stained with a PE-conjugated anti-human CD34 antibody, 7AAD, an FITC-conjugated anti-human CD45 antibody, and an APC-conjugated connexin 43 antibody. In addition, the remaining suspension was used to conduct a co-culture test with HUVECs (see (1) above). The co-culture experiment was conducted under a condition that an allophycocyanin (APC)-labeled vascular endothelial growth factor (VEGF, final concentration: 3 µg/mL) and umbilical cord blood mononuclear cells (cell count including 1 × 10⁴ CD34-positive cells) were added to HUVECs (1 × 10⁵ cells). The left portion of FIG. 5 shows a correlation between expression of connexin 43 in hematopoietic stem cells (CD34-positive, CD45-positive, and 7AAD-negative) and the VEGF uptake capability of HUVECs in the co-culture experiment. It was found that the expression intensity of connexin 43 as an indicator of VEGF uptake promotion capability is useful as a selectable marker for mononuclear cells having a high therapeutic effect.

### (5) Expression of CD18 in umbilical cord blood mononuclear cells before freezing and after thawing

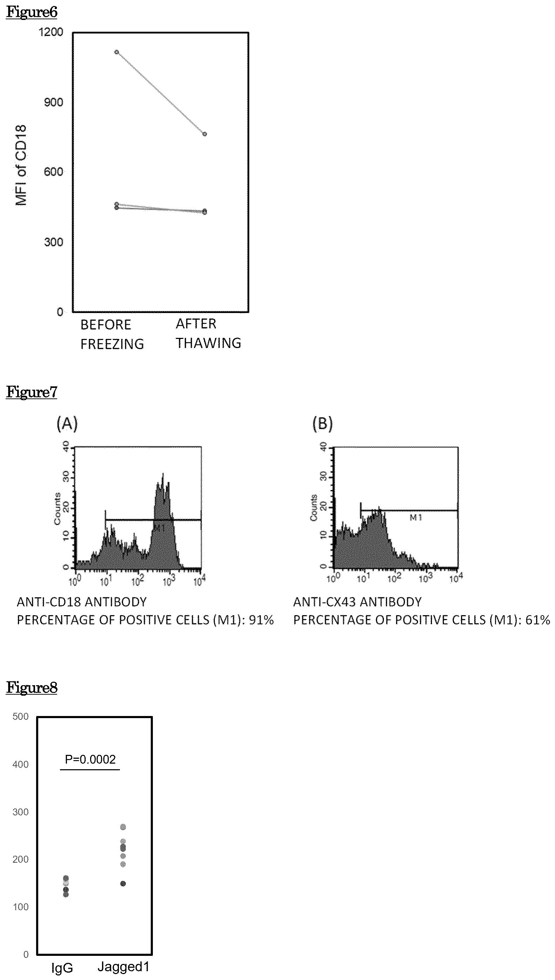

In fact, a study for expression of CD18 before freezing and after thawing of umbilical cord blood mononuclear cells showed that there were some umbilical cord blood mononuclear cells that underwent a large decrease in expression, and others that underwent almost no change in expression, as in the case of the heparin treatment. The former had high expression of CD18, while the latter had low expression of CD18 (FIG. 6).

Specifically, a mononuclear cell fraction was removed from umbilical cord blood by Ficoll-Paque specific gravity centrifugation described above, and was equally divided.

One of the two mononuclear cell fractions was stained with a PE-conjugated anti-human CD34 antibody, 7AAD, an FITC-conjugated anti-human CD45 antibody, and an APC-conjugated CD18 antibody. The expression level of a CD18 antigen in hematopoietic stem cells (CD34-positive, CD45-positive, and 7AAD-negative) was assessed using a FACS Canto II cell analyzer (BD Bioscience).

Next, a cryoprotection solution (cell banker) was added to the remaining mononuclear cell fraction, was frozen in a program freezer, and was stored in liquid nitrogen. After one week, the cryopreserved mononuclear cells were thawed, and the expression level of a CD18 antigen in hematopoietic stem cells was assessed using a technique similar to that described above.

As shown in FIG. 6, umbilical cord blood mononuclear cells having high expression of CD18 underwent a significant decrease in CD18 expression when those before freezing and after thawing were compared, while umbilical cord blood mononuclear cells having low expression of CD18 remained low before freezing and after thawing.

Next, to further confirm the usefulness of CD18-positive cells, the following study was conducted.

### (6) Collection of CD18-positive cells

The method for collecting cells having a high therapeutic effect according to the present invention had the following steps. Specifically, the method for collecting cells has: a collection step of collecting human bone marrow fluid with a diluent that does not contain heparin; and a separation step of adding a specific gravity centrifugal liquid that does not contain heparin to the collected human bone marrow fluid, and performing specific gravity centrifugation. In this example, the method was specifically carried out as follows. A 23-G needle was attached to a 2.5-ml syringe, and the syringe was filled with 2 ml of physiological saline. Both ends of a mouse femur were cut with surgical scissors. The needle was inserted into one end of the bone marrow cavity. The physiological saline was quickly injected to obtain bone marrow fluid that flowed out from the other end. An anticoagulant such as heparin is typically often used because bone marrow fluid coagulates easily. In the present invention, however, heparin was not used, and only dilution with physiological saline was used to inhibit coagulation of the bone marrow fluid.

The bone marrow fluid that was diluted with physiological saline to inhibit coagulation was placed on a specific gravity centrifugal liquid (Ficoll-premium, manufactured by GE), followed by centrifugation at 600g for 20 minutes. Thereafter, a cell group in the buffy coat layer was collected.

### (7) Test for CD18-positive cells

After collection of the cell group, surface marker analysis was conducted with FACS (BD FACSCalibur) using an anti-CD18 antibody.

Dead cells were excluded from the analysis using a 7AAD (7-amino-actinomycin D, manufactured by BD Bioscience, product number: 559925, dilution factor: 50x) reagent. The respective isotype controls were used as negative controls.

Concerning characteristics of the cell group, it was observed in analysis by flow cytometry that a CD18 antigen was expressed in at least 90% of the cells (FIG. 7). In the flow cytometric analysis, an antibody is directly or indirectly fluorescently labeled, and a target cell group is measured using a flow cytometer, to calculate the percentage of cells indicating a positive reaction. The positive reaction by flow cytometry is defined as a reaction in which the intensity of fluorescence at a particular wavelength emitted by a fluorophore used for labeling is within a range of fluorescent intensity higher than the intensity of the background measured without use of the primary antibody.

Similarly, after collection of the cell group, surface marker analysis was conducted with FACS (BD FACSCalibur) using an anti-Jagged1 antibody. It had been demonstrated that Jagged1 is expressed in CD34-positive cells (FIG. 8). Therefore, in order to study the regeneration promotion effect of the Jagged1 molecule, a Jagged1 peptide having Jagged1 activity (H-Cys-Asp-Asp-Tyr-Tyr-Tyr-Gly-Phe-Gly-Cys-Asn-Lys-Phe-Cys-Arg-Pro-Arg-OH) was added to a HUVEC culture medium. The VEGF uptake promotion effect of the Jagged1 peptide was studied.

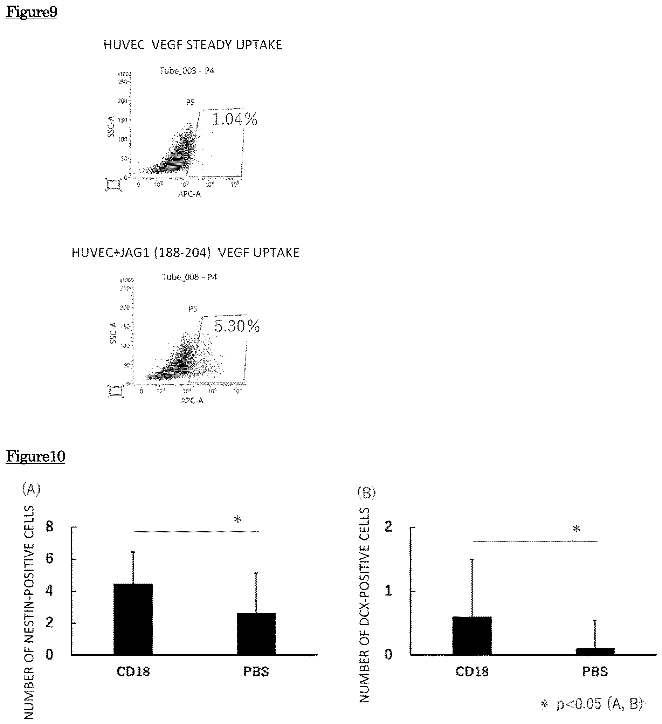

As a result, it was found that while 1.04% of the HUVECs took up VEGF in the absence of a stimulus, 5.30% of the HUVECs took up VEGF in the presence of a stimulus by Jagged1 (FIG. 9). These results show that expression of Jagged1 in stem cells is important to the regeneration promotion effect.

### (8) Administration of CD18-positive cells to aged mice

In 100 µl of a PBS, 1 × 10⁵ cells were suspended. The suspension was injected into CB-17 mice that were at least 80 weeks old (aged mice) through the tail vein every other day, a total of five times. The same amount of a PBS was intravenously injected into controls.

Twenty-four hours after the last intravenous administration, the brain was removed and fixed in 2% paraformaldehyde. Thereafter, the brain was sectioned into 20-µm sections using a vibratome (manufactured by Leica Camera AG). The sections were stained with an anti-nestin antibody (manufactured by Merck Millipore, product number: MAB5326, dilution factor: 1:200), an anti-doublecortin antibody (DCX; manufactured by Merck Millipore, product no. AB2253, dilution factor: 1:350), and DAPI for nuclear staining (manufactured by Thermo Fisher Scientific Inc., product no. D1306, dilution factor: 1: 1,000) (CD18 cell-administered mice: 7 mice, a total of 22 sections, PBS-administered mice: 6 mice, a total of 20 sections).

A confocal image was captured using a fluorescence microscope (BZ-X810, manufactured by Keyence Corporation) to assess newborn neuron marker (nestin)-positive cells in the granule cell layer of the hippocampal dentate gyrus and newborn neuron (DCX)-positive cells in the granule cell layer. In order to quantitatively assess the effect of CD18-positive cell administration, the number of each type of newborn neuron marker was counted. As a result, it was found that CD18-positive cell administration causes a statistically significant increase in both nestin-positive cells (FIG. 10A) and DCX-positive cells (FIG. 10B) per section.

In patients having diseases associated with age-related neurological dysfunction, neurogenesis is impaired in the hippocampus. According to the present invention, neurogenesis is promoted in the hippocampus, which allows prevention and/or treatment of such diseases. Even in the case in which neurological function is reduced due to aging, neurogenesis is promoted in the hippocampus according to the present invention, which allows improvement or slowing of aging.

### INDUSTRIAL APPLICABILITY

The present invention is useful in treatment of cerebral infarction and age-related neurological disorders.

## Claims

1. A cell preparation comprising:
CD18-positive cells.

2. The cell preparation of claim 1,
wherein
a glucose transporter (GLUT) is expressed in the CD18-positive cells.

3. The cell preparation of claim 2,
wherein
the glucose transporter is a class-I glucose transporter (GLUT1).

4. The cell preparation of claim 1,
wherein
a connexin is expressed in the CD18-positive cells.

5. The cell preparation of claim 4,
wherein
the connexin is connexin 43.

6. The cell preparation of claim 1,
wherein
Jagged1 is expressed in the CD18-positive cells.

7. The cell preparation of claim 1,
wherein
a glucose transporter (GLUT 1) and connexin 43 are expressed in the CD 18-positive cells.

8. The cell preparation of claim 1,
wherein
a glucose transporter (GLUT1), connexin 43, and Jagged1 are expressed in the CD18-positive cells.

9. The cell preparation of claim 1,
wherein
the CD18-positive cells are a stem cell.

10. The cell preparation of claim 9,
wherein
the stem cell is an adult stem cell.

11. The cell preparation of claim 10,
wherein
the adult stem cell is a hematopoietic stem cell.

12. The cell preparation of claim 11,
wherein
the hematopoietic stem cell is a CD34-positive bone marrow mononuclear cell.

13. The cell preparation of claim 11,
wherein
the hematopoietic stem cell is a CD34-positive umbilical cord blood mononuclear cell.

14. The cell preparation of any one of claims 1-13,
wherein
the cell preparation is a brain function improver, brain metabolism activator, brain circulation improver, neurotransmission function improver, brain protective agent, or memory disorder improver.

15. The cell preparation of any one of claims 1-14,
wherein
the CD18-positive cells are a cell group in which the percentage of cells exhibiting a CD18-positive reaction in analysis by flow cytometry is at least 90%.

16. An agent for promoting neurogenesis in a hippocampus, comprising:
CD18-positive cells.

17. The agent of claim 16,
wherein
the hippocampus is the hippocampal dentate gyrus.

18. The agent of claim 16 or 17,
wherein
the CD18-positive cells are connexin 43-positive.

19. The agent of claim 18,
wherein
the CD18-positive cells are a cell group in which the percentage of cells exhibiting a connxin 43-positive reaction in analysis by flow cytometry is at least 60%.

20. The agent of any one of claims 16-19,
wherein
the agent is used for prevention and/or treatment of an age-related decrease in neurological function.

21. The agent of any one of claims 16-19,
wherein
the agent is used for prevention and/or treatment of a disease associated with an age-related impairment of neurological dysfunction.

22. The agent of any one of claims 16-19,
wherein
the agent is used for prevention and/or treatment of a psychiatric disorder.

23. The agent of claim 22,
wherein
the psychiatric disorder is selected from any of depression, schizophrenia, bipolar disorder, or autism spectrum disorder.

24. The agent of any one of claims 16-19,
wherein
the agent is used for prevention and/or treatment of dementia.

25. The agent of claim 24,
wherein
the dementia is a neurodegenerative disease selected from any of Alzheimer's disease dementia, Lewy body dementia, frontotemporal dementia, corticobasal degeneration, progressive supranuclear palsy, or Parkinson's disease.

26. A cell collection method for collecting human bone marrow fluid and separating a human bone marrow fluid-derived cell group by specific gravity centrifugation, the method comprising:
collecting human bone marrow fluid with a diluent that does not contain heparin; and
adding a specific gravity centrifugal liquid that does not contain heparin to the collected human bone marrow fluid, and performing specific gravity centrifugation.
